# EUROPEAN PATENT APPLICATION

(11) **EP 4 520 342 A1**
(43) Date of publication of application: **12.03.2025**
(21) Application number: 23937792.2
(22) Date of filing: 31.08.2023
(51) Int. Cl.: A61K 35/747, A61P 21/00, A61P 39/06, A23L 33/135

(54) **USE OF LACTICASEIBACILLUS PARACASEI LC86 IN PREPARING PRODUCT USED FOR PREVENTING, RELIEVING OR TREATING SENILITY-RELATED MYATROPHY**

(30) Priority: 13.07.2023 CN 202310859888
(71) Applicant: Wecare Probiotics Co., Ltd., Suzhou, Jiangsu 215200 (CN)
(72) Inventor: FANG, Shuguang, Suzhou, Jiangsu 215200 (CN); GAI, Zhonghui, Suzhou, Jiangsu 215200 (CN); FAN, Yixuan, Suzhou, Jiangsu 215200 (CN); DONG, Yao, Suzhou, Jiangsu 215200 (CN); ZHU, Jianguo, Suzhou, Jiangsu 215200 (CN)
(74) Representative: Prinz & Partner mbB
(86) International application number: PCT/CN2023/116068
(87) International publication number: WO 2025/010811

(57) **Abstract**

Provided is a use of *Lacticaseibacillus paracasei* LC86 with the deposit number of CGMCC No. 1.12731 in the preparation of a product for preventing, alleviating, or treating aging-related muscle atrophy. The *Lacticaseibacillus paracasei* strain can significantly ameliorate aging-related muscle atrophy, which is specifically reflected by: (1) significantly decreasing the senescence score of SAMP mice; (2) significantly improving forelimb grip force and four-limb hanging ability of SAMP mice; (3) significantly increasing the level of gastrocnemius muscle glycogen, and decreasing the protein carbonyl content of gastrocnemius muscle; (4) decreasing the levels of pro-inflammatory cellular inflammatory factors TNF-α, IL-6, and MCP1 in mice serum, and increasing the level of anti-inflammatory cellular inflammatory factor IL-10 in serum; and (5) significantly increasing the mouse liver SOD enzyme activity, increasing the liver GSH content, increasing the liver CAT enzyme level, and decreasing the liver MDA level.

## Description

### TECHNICAL FIELD

The present application belongs to the field of microbial technology, relates to a novel use of *Lacticaseibacillus paracasei,* and specifically relates to a use of *Lacticaseibacillus paracasei* LC86 in the preparation of a product for preventing, alleviating, or treating aging-related muscle atrophy.

### BACKGROUND

With the increasingly prominent issues of aging and related diseases worldwide, sarcopenia is a type of disease commonly prevalent in old people, characterized by loss of muscle mass and function. It has been reported that more than 30% of people over 80 years of age are suffering from sarcopenia. This disease not only diminishes the life quality of old people, for example, heightens the risk of falls, disability, fractures, and death, but also increases the risk of quality declines of life. Therefore, maintaining muscle function has become an important factor in healthy aging. Research has indicated that the dysbiosis of gut microbiota is common among old people, which may lead to inflammatory response and metabolic resistance. The age-associated change composition in the gut microbiota may contribute to the sarcopenia of old people through the gut-muscle axis. More and more pieces of research evidence support the opinion that the muscle function is modulated by gut microbiota.

Lactobacilli, a type of probiotics, are widely distributed in the human intestinal tract and are considered to be one of the important members of maintaining the intestinal microecological balance. Their good adhesion facilitates the maintenance of intestinal flora structure, and effectively protects the integrity of morphology and metabolic function of the intestinal mucosa. The lactobacilli play an important role in preventing and treating the dysbiosis of gut microbiota, the main mechanism of which is achieved through the steric hindrance effect. Besides, probiotic lactobacilli can produce a variety of bacteriostatic components in the intestinal tract, such as acids, antimicrobial peptides, and bioenzymes, thereby suppressing pathogenic bacteria in growth and activity. Because the lactobacilli has the characteristics of safe and healthy, do not cause side effects, and can be colonized in animal bodies and exert lots of beneficial effects, the lactobacilli microecological preparations have been widely used in the field of clinical supplements.

One of the age-related statistics is muscle loss, including loss of muscle mass and loss of muscle function. Although the factors contributing to age-related muscle decline are not clear yet, some scientists believe that the factors such as oxidative stress and reactive oxygen might influence the cell signaling pathway, accelerate protein degradation, and impede protein synthesis in muscle fibers. Another explanation ascribes the muscle decline to the inflammatory cytokines IL-6 and TNF-α, which may induce muscle proteolysis. At present, there are no drugs that specifically target aging-related muscle atrophy. According to the pathogenesis and clinical approach of aging-related muscle atrophy, the strategies of anti-inflammatory cytokines, antioxidants, and intestinal flora modification can be selected to treat the aging-related muscle atrophy. Therefore, it is important to develop more probiotic products to alleviate the problem of aging-related muscle atrophy.

### SUMMARY

The present application provides use of *Lacticaseibacillus paracasei* LC86 in the preparation of a product for preventing, alleviating, or treating aging-related muscle atrophy.

In a first aspect, the present application provides a use of *Lacticaseibacillus paracasei* LC86 in the preparation of a product for preventing, alleviating, or treating aging-related muscle atrophy.

The *Lacticaseibacillus paracasei* is named as the *Lacticaseibacillus paracasei* LC86 strain, deposited in China General Microbiological Culture Collection Center on July 20, 2020, with the deposit number of CGMCC No. 1.12731, and the address is Court 1 (Room 3), West Beichen Road, Chaoyang District, Beijing.

In the present application, it is creatively found that the *Lacticaseibacillus paracasei* strain can significantly ameliorate aging-related muscle atrophy, which is specifically reflected by: (1) significantly decreasing the senescence score of SAMP mice; (2) significantly improving forelimb grip force and four-limb hanging ability of SAMP mice; (3) significantly increasing the level of gastrocnemius muscle glycogen, and decreasing the protein carbonyl content of gastrocnemius muscle; (4) decreasing the levels of pro-inflammatory cellular inflammatory factors TNF-α, IL-6, and MCP1 in mice serum, and increasing the level of anti-inflammatory cellular inflammatory factor IL-10 in serum; and (5) significantly increasing the mouse liver SOD enzyme activity, increasing the liver GSH content, increasing the liver CAT enzyme level, and decreasing the liver MDA level.

Preferably, in the product for preventing, alleviating, or treating aging-related muscle atrophy, the *Lacticaseibacillus paracasei* LC86 has a viable bacterial count of at least 1×10⁹ CFU/mL or 1×10⁹ CFU/g, which may be 1×10⁹ CFU/g (CFU/mL), 5×10⁹ CFU/g (CFU/mL), 1×10¹⁰ CFU/g (CFU/mL), 5×10¹⁰ CFU/g (CFU/mL), 1×10¹¹ CFU/g (CFU/mL), 5×10¹¹ CFU/g (CFU/mL), or 1×10¹² CFU/g (CFU/mL). Other specific point values within the above numerical range are all appropriate to be used and will not be redundantly recited herein.

In a second aspect, the present application provides a probiotic preparation for preventing, alleviating, or treating aging-related muscle atrophy, wherein a strain in the probiotic preparation comprises *Lacticaseibacillus paracasei* LC86.

Since the *Lacticaseibacillus paracasei* LC86 strain is a probiotic, it has high safety and is less prone to incurring resistance when used in the preparation of the product for preventing, alleviating, or treating aging-related muscle atrophy.

Preferably, the probiotic preparation for preventing, alleviating, or treating aging-related muscle atrophy further comprises *Bifidobacterium longum* BL21.

The *Bifidobacterium longum* BL21 strain is deposited in China General Microbiological Culture Collection Center on January 27, 2015, with the deposit number of CGMCC No. 10452, and the specific deposit address is Court 1 (Room 3), West Beichen Road, Chaoyang District, Beijing.

Since the *Bifidobacterium longum* strain BL21 strain is a probiotic, it has high safety and is less prone to incurring resistance when used in the preparation of the product for preventing, alleviating, or treating aging-related muscle atrophy.

In the present application, a novel probiotic combination method is creatively developed. The *Lacticaseibacillus paracasei* LC86 strain and *Bifidobacterium longum* BL21 strain are combined. It is found that those two strains have potential interactions, and can collaborate with each other to bring a synergistic efficacy on ameliorating the aging-related muscle atrophy. With the same amount of bacteria, compared with the single LC86 strain or single BL21 strain, the two-strain combination has significantly improved performance in the above-mentioned efficacies, obtaining unanticipated technical benefits for a person skilled in the art.

Preferably, a ratio of the viable bacterial count of the *Lacticaseibacillus paracasei* LC86 to the *Bifidobacterium longum* BL21 is (2-4): 1 in the probiotic preparation for preventing, alleviating, or treating aging-related muscle atrophy.

The ratio of the viable bacterial count of the *Lacticaseibacillus paracasei* LC86 to the *Bifidobacterium longum* BL21 in the probiotic preparation for preventing, alleviating, or treating aging-related muscle atrophy may be 2: 1, 3: 1, 7: 2, or 4: 1. Other specific point values within the above numerical range are all appropriate to be used and will not be redundantly recited herein.

In the probiotic preparation for preventing, alleviating, or treating aging-related muscle atrophy, the two strains of *Lacticaseibacillus paracasei* LC86 and *Bifidobacterium longum* BL21 have a better synergistic effect when they satisfy the above-mentioned specific ratio of viable bacterial counts.

Preferably, the probiotic preparation for preventing, alleviating, or treating aging-related muscle atrophy has the *Lacticaseibacillus paracasei* LC86 and the *Bifidobacterium longum* BL21 in a form of lyophilized powder.

Preferably, the lyophilized powder is prepared by a method comprising the steps of:
inoculating the *Lacticaseibacillus paracasei* LC86 and the *Bifidobacterium longum* BL21 respectively in culture media, culturing to obtain a bacterial solution, centrifuging, collecting a bacterial pellet, mixing with a lyoprotectant, and lyophilizing to obtain the lyophilized powder.

Preferably, the culture medium comprises glucose, peptone, beef extract powder, yeast extract powder, K₂HPO₄, diammonium hydrogen citrate, sodium acetate, MgSO₄, MnSO₄, or polysorbate 80.

Preferably, the components in the culture medium comprise (by the concentration) 20-40 g/L glucose, 10-15 g/L peptone, 5-8 g/L beef extract powder, 3-7 g/L yeast extract powder, 2-3 g/L K₂HPO₄, 2-3 g/L diammonium hydrogen citrate, 3-5 g/L sodium acetate, 0.3-0.5 g/L MgSO₄, 0.05-0.1 g/L MnSO₄, and 0.5-1.5 g/L polysorbate 80.

Specific values in the above 20-40 g/L are, for example, 20 g/L, 22 g/L, 25 g/L, 27 g/L, 30 g/L, 32 g/L, 35 g/L, 37 g/L, or 40 g/L.

Specific values in the above 10-15 g/L are, for example, 10 g/L, 11 g/L, 12 g/L, 13 g/L, 14 g/L, or 15 g/L.

Specific values in the above 5-8 g/L are, for example, 5 g/L, 5.5 g/L, 6 g/L, 6.5 g/L, 7 g/L, 7.5 g/L, or 8 g/L.

Specific values in the above 3-7 g/L are, for example, 3 g/L, 4 g/L, 5 g/L, 6 g/L, or 7 g/L.

Specific values in the above 2-3 g/L are, for example, 2 g/L, 2.2 g/L, 2.4 g/L, 2.6 g/L, 2.8 g/L, or 3 g/L.

Specific values in the above 3-5 g/L are, for example, 3 g/L, 3.2 g/L, 3.4 g/L, 3.6 g/L, 3.8 g/L, 3 g/L, 4.2 g/L, 4.4 g/L, 4.6 g/L, 4.8 g/L, or 5 g/L.

Specific values in the above 0.3-0.5 g/L are, for example, 0.3 g/L, 0.32 g/L, 0.35 g/L, 0.37 g/L, 0.4 g/L, 0.42 g/L, 0.45 g/L, 0.47 g/L, or 0.5 g/L.

Specific values in the above 0.05-0.1 g/L are, for example, 0.05 g/L, 0.06 g/L, 0.07 g/L, 0.08 g/L, 0.09 g/L, or 0.1 g/L.

Specific values in the above 0.5-1.5 g/L are, for example, 0.5 g/L, 0.6 g/L, 0.7 g/L, 0.8 g/L, 0.9 g/L, 1 g/L, 1.1 g/L, 1.2 g/L, 1.3 g/L, 1.4 g/L, or 1.5 g/L.

Preferably, the culture is performed at a temperature of 30-40°C, and the culture is performed for a period of 12-48 h.

The temperature of the culture may be 30°C, 31°C, 32°C, 33°C, 34°C, 35°C, 36°C, 37°C, 38°C, 39°C, or 40°C, and other specific point values within the above numerical range are all appropriate to be used and will not be redundantly recited herein.

The period of the culture may be 12 h, 14 h, 16 h, 18 h, 20 h, 22 h, 24 h, 30 h, 36 h, 40 h, or 48 h, and other specific point values within the above numerical range are all appropriate to be used and will not be redundantly recited herein.

Preferably, the lyoprotectant comprises any one or a combination of at least two of trehalose, sucrose, lactose, skim milk powder, glycerol, mannitol, or polysorbate. The combination of at least two is, for example, a combination of trehalose and sucrose, a combination of mannitol and polysorbate, a combination of sucrose and mannitol, or any other combinations.

Preferably, the probiotic preparation for preventing, alleviating, or treating aging-related muscle atrophy further comprises a functional additive.

Preferably, the functional additive comprises any one or a combination of at least two of inulin, resistant dextrin, fructo-oligosaccharide, isomalto-oligosaccharide, or galacto-oligosaccharide.

The combination of at least two is, for example, a combination of inulin and resistant dextrin, a combination of fructo-oligosaccharide and isomalto-oligosaccharide, a combination of fructooligosaccharide and galactooligosaccharide, or any other combinations.

In a third aspect, the present application provides a use of the probiotic preparation as described in the second aspect in the preparation of a product for preventing, alleviating, or treating aging-related muscle atrophy.

Preferably, the product comprises a nutraceutical or a pharmaceutical product.

Preferably, a dosage form of the nutraceutical or the pharmaceutical product comprises liquids, tablets, capsules, or granules.

Preferably, the pharmaceutical product further comprises a pharmaceutical carrier and/or a pharmaceutically acceptable adjuvant.

Preferably, the pharmaceutically acceptable adjuvant comprises any one or a combination of at least two of an excipient, a filler, a disintegrant, a binder, a lubricant, a diluent, or a flavor.

The combination of at least two is, for example, a combination of an excipient and a filler, a combination of a filler and a disintegrant, and a combination of a disintegrant and a binder, and any other combination is appropriate and will not be redundantly recited herein.

In a fourth aspect, the present application provides a use of *Lacticaseibacillus paracasei* LC86 in the preparation of a TNF-a antagonist, an IL-6 antagonist, an MCP-1 antagonist, an SOD enzyme promoter, a GSH promoter, a CAT enzyme promoter, an MDA antagonist, or an IL-10 secretion promoter.

Compared with the prior art, the present application has the following beneficial effects:
The present application provides a use of *Lacticaseibacillus paracasei* LC86 in the preparation of a product for preventing, alleviating, or treating aging-related muscle atrophy. The *Lacticaseibacillus paracasei* strain can significantly ameliorate aging-related muscle atrophy, which is specifically reflected by: (1) significantly decreasing the senescence score of SAMP mice; (2) significantly improving forelimb grip force and four-limb hanging ability of SAMP mice; (3) significantly increasing the level of gastrocnemius muscle glycogen, and decreasing the protein carbonyl content of gastrocnemius muscle; (4) decreasing the levels of pro-inflammatory cellular inflammatory factors TNF-α, IL-6, and MCP1 in mice serum, and increasing the level of anti-inflammatory cellular inflammatory factor IL-10 in serum; and (5) significantly increasing the mouse liver SOD enzyme activity, increasing the liver GSH content, increasing the liver CAT enzyme level, and decreasing the liver MDA level.

### BRIEF DESCRIPTION OF DRAWINGS

FIG. 1 shows changes in senescence scores of the mice in each group.
FIG. 2 is statistical graphs showing the results of muscle strength tests for the mice in each group, including a forelimb grip test (A) and a four-limb hanging test (B).
FIG. 3 is statistical graphs showing the results of colonic gastrocnemius muscle glycogen (A) and protein carbonyl (B) content determination for the mice in each group.
FIG. 4 is statistical graphs showing the contents of tumor necrosis factor TNF-α (A), interleukin 6 (IL-6) (B), MCP1 (C), and interleukin 10 (IL-10) (D) in serum of the mice in each group.
FIG. 5 is statistical graphs showing the SOD enzyme activity (A), GSH level (B), CAT enzyme activity (C), and MDA content (D) in liver tissues of the mice in each group.

In each of the graph aforementioned, the bar charts having different lowercase letters indicate significant differences between the two groups (p<0.05).

### DETAILED DESCRIPTION

The technical solutions of the present application are further described below in terms of the drawings and specific embodiments. It should be clear to those skilled in the art that the embodiments are merely used for a better understanding of the present application and should not be regarded as a specific limitation to the present application.

Sources of part of the materials used in the examples and comparative examples are described below.

Formulation to prepare the MRS culture medium involved in the examples is as follows:
Calculated by 1000 mL of water, 10 g/L of peptone, 10 g/L of beef paste, 20 g/L of glucose, 2 g/L of sodium acetate, 5 g/L of yeast powder, 2 g/L of diammonium hydrogen citrate, 2.6 g/L of K₂PO₄·3H₂O, 0.1 g/L of MgSO₄·7H₂O, 0.05 g/L of MnSO₄, 1 mL/L of polysorbate 80, and 0.5 g/L cysteine hydrochloride. In those materials, the peptone, beef paste, glucose, sodium acetate, yeast powder, diammonium hydrogen citrate, K₂PO₄·3H₂O, MgSO₄·7H₂O, MnSO₄, polysorbate 80, and cysteine hydrochloride were purchased from Sinopharm Chemical Reagent Co., Ltd.

The test animals were SPF male SAMP8 mice, and the mouse feed was purchased from Shanghai SLAC Co., Ltd.; the levels of TNF-α, IL-6, MCP1, and IL-10 were determined by ELISA kits purchased from Shanghai Enzyme-linked Biotechnology Co., Ltd.; the levels of superoxide dismutase (SOD), glutathione (GSH), catalase (CAT), and malondialdehyde (MDA) were determined by test kits purchased from Nanjing Jiancheng Bioengineering Co., Ltd.

The *Lacticaseibacillus paracasei* LC86 strain involved in the examples is deposited in China General Microbiological Culture Collection Center on July 20, 2020, with the deposit number of CGMCC No. 1.12731, and the address is Court 1 (Room 3), West Beichen Road, Chaoyang District, Beijing.

The *Bifidobacterium longum* BL21 strain involved in the examples is deposited in China General Microbiological Culture Collection Center on January 27, 2015, with the deposit number of CGMCC No. 10452, and the specific deposit address is Court 1 (Room 3), West Beichen Road, Chaoyang District, Beijing.

Method for preparing lyophilized powder of *Lacticaseibacillus paracasei* LC86 involved in the examples:
the *Lacticaseibacillus paracasei* LC86 was inoculated onto the culture medium at a dose of 3% relative to the total mass of the MRS medium, and cultured at 37°C for 18 h to obtain a culture solution; the culture solution was centrifuged to obtain a bacterial pellet; the bacterial pellet were resuspended (at a mass ratio of the lyoprotectant to the bacterial pellet of 2: 1) with a trehalose lyoprotectant (containing 100 g/L of trehalose, 100 g/L of skim milk powder, and water as the solvent) to obtain a resuspending solution; the resuspending solution was lyophilized by the vacuum freezing method to obtain the lyophilized powder of *Lacticaseibacillus paracasei* LC86; the viable bacterial count of the lyophilized powder was tested to be 300 billion CFU/g.

Method for preparing a lyophilized powder of *Bifidobacterium longum* BL21 involved in the examples:
the *Bifidobacterium longum* BL21 was inoculated onto the culture medium at a dose of 3% relative to the total mass of the MRS medium, and cultured at 37°C for 18 h to obtain a culture solution; the culture solution was centrifuged to obtain a bacterial pellet; the bacterial pellet were resuspended (at a mass ratio of the lyoprotectant to the bacterial pellet of 2: 1) with a trehalose lyoprotectant (containing 100 g/L of trehalose, 100 g/L of skim milk powder, and water as the solvent) to obtain a resuspending solution; the resuspending solution was lyophilized by the vacuum freezing method to obtain the lyophilized powder of *Bifidobacterium longum* BL21; the viable bacterial count of the lyophilized powder was tested to be 300 billion CFU/g.

### Example 1

This example provides five probiotic preparations as follows:
(S1) the LC86 lyophilized powder preparation prepared above;
(S2) the BL21 lyophilized powder preparation prepared above;
(S3) an LC86+BL21 composite probiotic preparation, which was obtained by evenly mixing the LC86 lyophilized powder preparation with the BL21 lyophilized powder preparation at a ratio of the viable bacterial count of 4: 1;
(S4) an LC86+ *Bifidobacterium longum* ATCC 15707 composite probiotic preparation, which was obtained by evenly mixing the LC86 lyophilized powder preparation with the ATCC 15707 lyophilized powder preparation at a ratio of the viable bacterial count of 4: 1; and
(S5) an LC86+BL21+inulin composite probiotic preparation, which was obtained was mixing the LC86 lyophilized powder preparation, BL21 lyophilized powder preparation, and inulin, wherein a ratio of the viable bacterial count of the LC86 strain to the BL21 strain was 4: 1, and a mass ratio of the total mass of the probiotic lyophilized powder to the inulin was 1: 5.

### Example 2

This example explores the influence of the five probiotic preparations (S1, S2, S3, S4, and S5) prepared in Example 1 on the serum AST and ALT, triglyceride in serum and liver, oxidative stress marker content, pro-inflammatory cytokines in serum, and intestinal permeability for mice having aging-related muscle atrophy:

### (1) Animal grouping

Sixty 16-week-old male adult SAMP8 mice (with a body weight of 22±2 g) were randomly divided into six groups, 10 mice for each group; model group (daily saline gavage for the CTL group), probiotic preparation S1 group (given an LC86 bacterial dose of 1×10⁹ CFU/day), probiotic preparation S2 group (given a BL21 bacterial dose of 1×10⁹ CFU/day), probiotic preparation S3 group (given a total bacterial dose of LC86 and BL21 of 1×10⁹ CFU/day), probiotic preparation S4 group (given a total bacterial dose of LC86 and ATCC 15707 of 1×10⁹ CFU/day), and probiotic preparation S5 group (given a total bacterial dose of LC86 and BL21 of 1×10⁹ CFU/day), for 12 weeks. All mice were free of specific pathogens; ten 16-week-old young male SAMP8 mice (with a body weight of 22±2 g) were grouped as a blank control (youth group). The mice were kept individually in cages in a clean and quiet environment at a temperature of 23-25°C and a humidity of 50-70%, and the mice in each group had free access to food and water during the experimental period.

### (2) Blood sample collection:

After the 12-week experiment was completed, all mice were anesthetized with 10% chloral hydrate (2 mL/kg) by intraperitoneal injection and subjected to cervical dislocation, and blood samples, liver, and colon tissues were collected.

### (3) Assessment of senescence

The aging degree of the mice in each of the above S1, S2, S3, S4, S5, and CTL groups, and the aging degree of the 16-week-old young male SAMP8 mice (youth group) were assessed by a graded scoring system. Each item has five levels which respectively scores 0, 1, 2, 3, and 4; a higher score indicates a higher degree of aging. Indicators used in the assessment include reactive behavior, passive behavior, glossiness, coarseness, loss of hair, skin ulcers, periophthalmic lesions, and spinal lordosis and kyphosis. Scores for each indicator are added up to obtain a total score for each mouse. The scoring criteria for mouse senescence are shown in Table 1.

**Table 1**

| **Item** | **Definition** | **Grade 0** | **Grade 1** | **Grade 2** | **Grade 3** | **Grade 4** |
|---|---|---|---|---|---|---|
| **I. Behavior** | | | | | | |
| 1. Reactivity | The most intensive exploratory response observed within 30 seconds | Natural behavio r | A. Abnormal gait with no lessening of agility and behavior patterns B. Restlessnes s | Definite decrease in agility and behavior patterns | Does not move voluntarily but will move if nudged | Immobile |
| 2. Passivity | Escape reaction from pinching of the nuchal skin or from hanging by the forelimb | Natural escape reaction to plnchin g | Decrease in escape reaction | Loss of escape reaction to pinching. Preserved the righting reaction to manual turn over | Neither escape reaction to pinching nor righting reaction. Escape reaction to hanging by the forelimb | Escape reaction nil |

| **II. Appearance** | | | | | | |
|---|---|---|---|---|---|---|
| **1.Skin and hair** | | | | | | |
| (1) Glossiness | Glossiness | Natural gloss | Decrease in gloss | Complete disappearanc e of gloss | Complete disappeara nce of gloss and hair appears dirty | Complete disappeara nce of gloss and hair looks very dirty |
| (2) Coarseness | Coarseness of hair on the head, nucha, and dorsum, determined according to the number of palpable, fine clumps of hair | No coarsen ess | Coarseness of less than an area of the head | Coarseness of less than doubles the area of the head | Coarseness of less than 3 times areas of the head | Complete disappeara nce of gloss and hair looks very dirty |
| (3) Loss of hair | Loss or thinning of hair on the head, nucha, and dorsum, except for changes due to ulcer or periophthalmi c lesions | Neither loss nor thinning of hair | A. Loss of hair in less than an area of the head B. Thinning of hair in less than 1/2 of the area | A. Loss of hair in over one area of the head, less than in 1/4 of total area B. Thinning of hair in more than 1/2 of total area | Loss of hair in more than 1/4, in less than 1/2 of total area | Loss of hair in over 1/2 of total area |
| (4) Skin ulcers | Ulcer or healed ulcer on entire skin except for changes associated with periophthalmi c lesions | No evidenc e of ulcer | Healed ulcer or ulcer with scab | Ulcer without healing tendency, in less than one area of the head | Ulcer without healing tendency in more than one area of the head, in less than 1/4 area of all the skin | Ulcer without healing tendency in more than 1/4 area of whole skin |

| **2.Eyes** | | | | | | |
|---|---|---|---|---|---|---|
| (1) Periophthal mic Lesions | Catarrhal changes in the periophthalmi c area or swelling of the palpebra | No changes | Catarrhal changes limited to periophthal mic area or swelling of palpebra | Catarrhal changes extending to nose | Diarrhea changes extending further | |
| (2) Corneal pacity | Opaque changes of cornea with rough surface by direct ophthalmosco py | No opacity | Opacity with visible iris | Opacity with visible iris. Positive retinal reflex by transillumina tion | Opacity of entire cornea | |
| (3) Ulcer of the comes | Opaque changes of cornea with rough surface by direct ophthalmosco py | No ulcer | Linear ulcer correspondi ng to palpebral fissure | Extension of ulcer over most of the area | Ulcer of entire cornea | |
| (4) Cataract | Opaque changes of crystalline lens without retinal reflex by transilluminat ion. Impossible to soccer because of coexistence of grade 3 corneal opacity or ulcer | Natural reflectio n | Diminished reflection | No reflection | | |

| **3. Spine** | | | | | | |
|---|---|---|---|---|---|---|
| (1) kyphotic deformity | Examined by inspection and palpation | Natural anterior and posterio r curvatur e | Increased curvature disappears with digital pressure on the dorsum | Increased curvature disappears with a combination of manual cephalocauda l traction and digital pressure on the dorsum | Permanent curvature | |

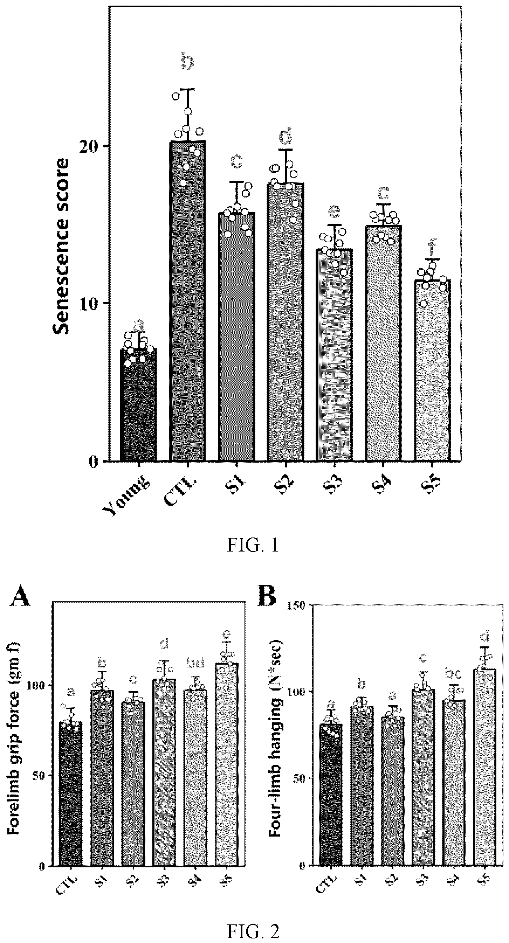

As shown in FIG. 1, the senescence score is significantly higher in the model group (CTL) compared to young mice. Compared with the model group (CTL), the senescence score of mouse serum is significantly reduced after the intervention of single-strain probiotic preparation S1. In addition, the senescence score of the composite probiotic preparation S3 group is lower than that of the single-strain probiotic preparation S1, and lower than that of the S4 group which has ATCC15707 compounded with, suggesting that there is a synergistic effect between *Lacticaseibacillus paracasei* LC86 and *Bifidobacterium longum* BL21 in slowing down the degree of aging. The senescence score of the composite probiotic preparation S5 group shows a further decrease compared to the composite probiotic preparation S3 group, indicating that the addition of inulin has a significant improving effect on the probiotic preparation.

### (4) Effects on muscle strength of mice:

The muscle strength of mice is assessed by the forelimb grip test and four-limb hanging test.

Forelimb grip test method: the mice in each group gripped a horizontal bar with their forelimbs while the use of restraint devices at the tail prohibits the use of their hindlimbs. The maximum grip force was recorded over 10 trials, and grip force values were expressed as grip force (gm f).

Four-limb hanging test method: mice in each group were placed upside down in the top of metal wire cages, and then mice will hang for a few seconds. The time that the mice were able to remain suspended was recorded. The test was repeated three times and the time was averaged for each mouse. The results were expressed as the holding impulse time (weight (g)×holding time (s)).

Sarcopenia is defined as the progressive loss of skeletal muscle mass and strength. This experiment evaluates the effects of each probiotic preparation on muscle strength in mice. The above methods are adopted to assess the muscle strength of mice in each group by the forelimb grip test and four-limb hanging test. Compared with the model group (CTL), the muscle strength of mice is increased after the intervention of single-strain probiotic preparation S1; in addition, the muscle strength of mice in the composite probiotic preparation S3 group is significantly higher than that of the single-strain probiotic preparation S1, and also higher than that of the S4 group which has ATCC 15707 compounded with, suggesting that there is a synergistic effect between *Lacticaseibacillus paracasei* LC86 and *Bifidobacterium longum* BL21 in slowing down the age-associated sarcopenia. The muscle strength of the composite probiotic preparation S5 group is stronger compared to the composite probiotic preparation S3 group, indicating that the addition of inulin has a significant improving effect for the LC86-cotaining probiotic preparation in preventing, alleviating, or treating aging-related muscle atrophy in mice.

### (5) Effect on muscle glycogen content and protein carbonyl content in gastrocnemius muscle

Assay method for colonic muscle glycogen: 100 mg of muscle tissue was homogenized in 0.5 mL of cold perchloric acid and then centrifuged at 15000 × g and 4°C for 15 min, and the supernatant was collected. Muscle glycogen content (expressed as mg/g muscle) was determined using a muscle glycogen assay kit (Nanjing Jiancheng Bioengineering Co., Ltd.).

Assay method for protein carbonyl content in colon gastrocnemius muscle: the concentration of protein carbonyl in mouse gastrocnemius muscle was determined by a protein carbonyl assay kit. The specific steps were as follows: 100 mg of gastrocnemius muscle sample was homogenized in phosphate buffer at pH = 7, and then centrifuged at 10000×g and 4°C for 10 min to collect the supernatant. The supernatant was incubated with dinitrophenylhydrazine for 1 h at room temperature. Subsequently, the protein was precipitated with trichloroacetic acid and resuspended in guanidine hydrochloride. After centrifugation at 10000×g and 4°C for 10 min, the absorbance of the supernatant was measured at a wavelength of 370 nm.

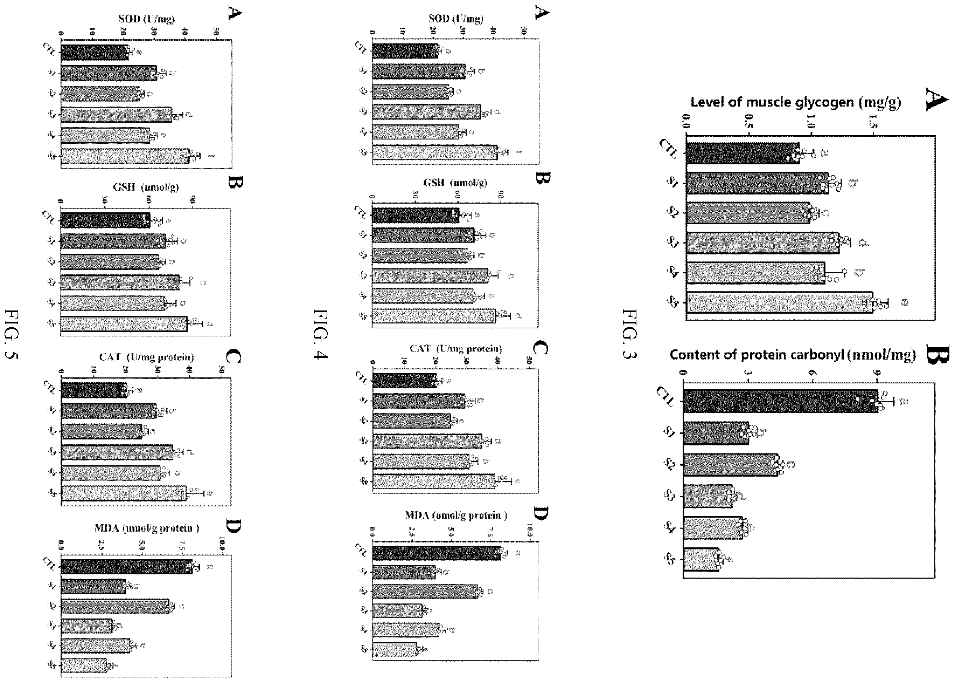

The results are shown in FIG. 3. Compared with the CTL group, the muscle glycogen level of mice is increased after the intervention of single-strain probiotic preparation S1. In addition, the muscle glycogen level of the composite probiotic preparation S3 group is significantly higher than that of the single-strain probiotic preparation S1, and also higher than that of the S4 group which has the *Bifidobacterium longum* ATCC 15707 compounded with, suggesting that there is a synergistic effect between *Lacticaseibacillus paracasei* LC86 and *Bifidobacterium longum* BL21 in increasing the muscle glycogen level of mice. The muscle glycogen level of the composite probiotic preparation S5 group is higher compared to the composite probiotic preparation S3 group, indicating that the addition of inulin has a significant improving effect for the LC86-cotaining probiotic preparation in increasing the muscle glycogen level of mice.

(7) Determination of serum tumor necrosis factors TNF-α, interleukin 6 (IL-6), MCP1, and IL-10 by the enzyme-linked immunosorbent assay (ELISA) in each group of mice:
The results are shown in FIG. 4. After the intervention of single-strain probiotic preparation S1, compared with the CTL group, the levels of TNF-α, interleukin 6 (IL-6), and MCP1 are decreased, while the level of interleukin 10 (IL-10) is increased in mice serum. In addition, the level of interleukin 10 (IL-10) in the composite probiotic preparation S3 group is significantly higher than that of the single-strain probiotic preparation S1, and also higher than that of the S4 group which has *Bifidobacterium longum* ATCC 15707 compounded with; the levels of TNF-α, interleukin 6 (IL-6), and MCP1 in the composite probiotic preparation S3 group have more significant reduction in mice serum compared with the S1 group, and the reduction is also more significant than the S4 group which has *Bifidobacterium longum* ATCC 15707 compounded with; the results indicate that there is a synergistic effect between *Lacticaseibacillus paracasei* LC86 and *Bifidobacterium longum* BL21 in decreasing the levels of TNF-α, interleukin 6 (IL-6), and MCP1, and increasing the level of interleukin 10 (IL-10). Compared with the composite probiotic preparation S3 group, the composite probiotic preparation S5 group has more significant reduction of levels of TNF-α, interleukin 6 (IL-6), and MCP1, and more significant increase of interleukin 10 (IL-10) level in mice serum, indicating that the addition of inulin has a significant improving effect on the probiotic preparation.

### (8) Determination of oxidative stress markers:

Mice were killed after the experiment was completed; the colon tissues of mice in each group were added to normal saline and then homogenized to obtain 10% colon homogenate. The homogenate was centrifuged for 10 min (5000×g, 4°C) to obtain the supernatant. The levels of superoxide dismutase (SOD), glutathione (GSH), catalase (CAT), and malondialdehyde (MDA) were determined by a Nan assay kit.

The results are shown in FIG. 5: A represents superoxide dismutase (SOD), B represents glutathione (GSH), C represents catalase (CAT), and D represents malondialdehyde (MDA) level.

Compared with the CTL group, after the intervention of single-strain probiotic preparation S1, the SOD, GSH, and CAT levels of mice are increased, and the MDA level is decreased. The SOD, GSH, and CAT levels of the composite probiotic preparation S3 group are significantly increased which is better than the single-strain probiotic preparation S1, while the MDA level is significantly decreased compared with the S1; the SOD, GSH, and CAT levels are higher than those in the S4 group which has *Bifidobacterium longum* ATCC 15707 compounded with, and the MDA level is lower than the S4 group, indicating that the combination of *Lacticaseibacillus paracasei* LC86 and *Bifidobacterium longum* BL21 at a specific ratio has a synergistic effect in increasing the SOD, GSH, and CAT levels, and decreasing the MDA level in mice. Compared with the composite probiotic preparation S3 group, the composite probiotic preparation S5 group has higher SOD, GSH, and CAT levels, and lower MDA level, indicating that the addition of inulin has a significant improving effect in increasing the SOD, GSH, and CAT levels and decreasing the MDA level in mice.

The applicant has stated that although the detailed process of the present application is described through the above embodiments, the present application is not limited to the above-detailed process, which means that the implementation of the present application does not necessarily depend on the above-detailed process. It should be apparent to those skilled in the art that any improvements made to the present application, equivalent substitutions of various raw materials of the product, the addition of adjuvant ingredients, the selection of specific methods, etc., all fall within the protection scope and the disclosure scope of the present application.

## Claims

1. Use of *Lacticaseibacillus paracasei* LC86 in the preparation of a product for preventing, alleviating, or treating aging-related muscle atrophy;
wherein the *Lacticaseibacillus paracasei* LC86 is a *Lacticaseibacillus paracasei* LC86 strain with the deposit number of CGMCC No. 1.12731.

2. The use according to claim 1, wherein in the product for preventing, alleviating, or treating aging-related muscle atrophy, the *Lacticaseibacillus paracasei* LC86 has a viable bacterial count of at least 1×10⁹ CFU/mL or 1×10⁹ CFU/g.

3. A probiotic preparation for preventing, alleviating, or treating aging-related muscle atrophy, wherein a strain in the probiotic preparation comprises *Lacticaseibacillus paracasei* LC86;
wherein the *Lacticaseibacillus paracasei* LC86 is a *Lacticaseibacillus paracasei* LC86 strain with the deposit number of CGMCC No. 1.12731.

4. The probiotic preparation for preventing, alleviating, or treating aging-related muscle atrophy according to claim 3, wherein the probiotic preparation further comprises *Bifidobacterium longum* BL21;
wherein the *Bifidobacterium longum* BL21 is a *Bifidobacterium longum* BL21 strain with the deposit number of CGMCC No. 10452.

5. The probiotic preparation for preventing, alleviating, or treating aging-related muscle atrophy according to claim 3 or 4, wherein a ratio of the viable bacterial count of the *Lacticaseibacillus paracasei* LC86 to the *Bifidobacterium longum* BL21 is (2-4): 1 in the probiotic preparation.

6. The probiotic preparation for preventing, alleviating, or treating aging-related muscle atrophy according to any one of claims 3-5, wherein the *Lacticaseibacillus paracasei* LC86 and the *Bifidobacterium longum* BL21 exists in a form of lyophilized powder in the probiotic preparation;
preferably, the lyophilized powder is prepared by a method comprising the steps of:
inoculating the *Lacticaseibacillus paracasei* LC86 and the *Bifidobacterium longum* BL21 respectively in culture media, culturing to obtain a bacterial solution, centrifuging, collecting a bacterial pellet, mixing with a lyoprotectant, and lyophilizing to obtain the lyophilized powder;
preferably, the culture is performed at a temperature of 30-40°C, and the culture is performed for a period of 12-48 h;
preferably, the lyoprotectant comprises any one or a combination of at least two of trehalose, sucrose, lactose, skim milk powder, glycerol, mannitol, or polysorbate.

7. The probiotic preparation for preventing, alleviating, or treating aging-related muscle atrophy according to any one of claims 3-6, wherein the probiotic preparation further comprises a functional additive;
preferably, the functional additive comprises any one or a combination of at least two of inulin, resistant dextrin, fructo-oligosaccharide, isomalto-oligosaccharide, or galacto-oligosaccharide.

8. Use of the probiotic preparation according to any one of claims 3-7 in the preparation of a product for preventing, alleviating, or treating aging-related muscle atrophy.

9. The use according to claim 8, wherein the product comprises a nutraceutical or a pharmaceutical product;
preferably, a dosage form of the nutraceutical or the pharmaceutical product comprises liquids, powders, tablets, capsules, or granules;
preferably, the pharmaceutical product further comprises a pharmaceutical carrier and/or a pharmaceutically acceptable adjuvant;
preferably, the pharmaceutically acceptable adjuvant comprises any one or a combination of at least two of an excipient, a filler, a disintegrant, a binder, a lubricant, a diluent, or a flavor.

10. Use of *Lacticaseibacillus paracasei* LC86 in the preparation of a TNF-a antagonist, an IL-6 antagonist, an MCP-1 antagonist, an SOD enzyme promoter, a GSH promoter, a CAT enzyme promoter, an MDA antagonist, or an IL-10 secretion promoter.
